# EUROPEAN PATENT APPLICATION

(11) **EP 4 529 879 A2**
(43) Date of publication of application: **02.04.2025**
(21) Application number: 24200828.2
(22) Date of filing: 17.09.2024
(51) Int. Cl.: A61B 18/18

(54) **MICROWAVE ABLATION PROBE ASSEMBLY AND CABLE STRUCTURE**

(30) Priority: 28.09.2023 US 202318477374
(71) Applicant: Varian Medical Systems, Inc., Palo Alto, CA 94304 (US)
(72) Inventor: HATTAN, Paul, Minneapolis, 55401 (US); MAY, Justin, Austin, 78738 (US); FALLAHI, Hojjatollah, Minneapolis, 55401 (US)
(74) Representative: Mathisen & Macara LLP

(57) **Abstract**

A cable bundle for a microwave ablation probe assembly may include a cable configured to deliver a microwave signal from a microwave generator to an antenna in a probe and a coolant conduit positioned adjacent the cable. The cable bundle may also include a conductive layer positioned externally to the cable and the coolant conduit.

## Description

### FIELD

The present disclosure relates to microwave ablation probe assemblies and cable structures that may be used in connection with microwave ablation probe assemblies.

### BACKGROUND

This section provides background information related to the present disclosure which is not necessarily prior art.

Ablation apparatuses and systems are used to perform ablation treatments in which a probe is inserted at or near a target tissue in a patient. The target tissue is typically an abnormal tissue such as a tumor or other growth. The ablation treatment is performed to destroy the target tissue to reduce the likelihood of further growth or spreading of the target tissue in the patient. One type of ablation is thermal ablation. In thermal ablation, the probe that is positioned at or near the target tissue causes the temperature of the target tissue, typically in a localized region proximate the probe, to be elevated to a temperature at which the target tissue is destroyed.

The thermal ablation can be induced by the emission of microwaves at or near the target tissue. A microwave generator may be used that sends a microwave signal to an antenna in the ablation probe. The antenna may emit microwave energy into surrounding tissue causing the temperature of such tissue to be elevated to temperatures sufficient to cause thermal ablation. Microwave ablation probes are typically connected to the ablation probe via a cable or wire that electrically couples the microwave generator to the ablation probe.

Existing microwave ablation probes and related cables or wires suffer from various drawbacks. For example, existing ablation probes and related cables can overheat due to the electrical signal being sent from the microwave generator to the probe through the cable. The excess heat can make the cables and/or probes difficult to handle and/or come into contact with during an ablation treatment. The excess heat can cause a burn or other hazards to users during operation. In other examples, existing cables and/or probes may be difficult to manipulate by a user due to the size of the cable and/or the rigidity of the cable. Such existing cables can be difficult to bend and/or otherwise route to a desired location to perform an ablation treatment. There exists a need, therefore, for improved ablation probes and cabling structures that can be easily operated and maintain an operating temperature at or below acceptable levels.

### SUMMARY

This section provides a general summary of the disclosure, and is not a comprehensive disclosure of its full scope or all of its features.

In a first aspect, the present invention provides a cable bundle comprising a cable configured to deliver a microwave signal from a microwave generator to an antenna; a coolant conduit positioned adjacent the cable; a conductive layer positioned externally to the cable and the coolant conduit.

In one aspect, the conductive layer is in thermal contact with both the cable and the coolant conduit to conduct thermal to energy from the cable to the coolant conduit.

In another aspect, the conductive layer may conduct thermal energy from the warmest radial and/or axial location of the cable bundle to cooler radial and/or axial locations of the cable bundle more effectively than other cable bundle materials, thereby reducing the maximum surface temperature of the cable bundle.

In another aspect, the coolant conduit is configured to guide a flow of coolant through the cable bundle.

In another aspect, the coolant conduit comprises a first coolant conduit and the cable bundle further comprises a second coolant conduit, the first coolant conduit configured to guide coolant toward the antenna and the second coolant conduit configured to guide coolant away from the antenna.

In another aspect, the first coolant conduit, the second coolant conduit, and the cable contact each other and contact the conductive layer.

In another aspect, the conductive layer comprises a plurality of braided fibers.

In another aspect, the plurality of braided fibers comprises aluminum or copper fibers.

In another aspect, the cable bundle further comprises one or more low-voltage wires positioned inside the conductive layer.

In another aspect, the cable bundle further comprises an outer layer positioned externally to the conductive layer. The outer layer may include an insulating material.

In another aspect, the cable bundle further comprises a filler material positioned in one or more gaps between the coolant conduit and the conductive layer.

In another aspect, the cable bundle further comprises a console connector configured to electrically couple the cable to a microwave generator in a microwave ablation console.

In another aspect, the coolant conduit is fluidly connected to a coolant cartridge configured to be received in a coolant port of a microwave ablation console.

In another aspect, the cable bundle may have an axial length of at least 36 inches in length.

In a second aspect, the present invention provides a microwave ablation probe assembly comprising a handle configured to operably connect to a cable bundle comprising a power cable, a coolant supply conduit and a coolant return conduit; a needle connected to the handle and extending away from the handle to a distal end; and a microwave antenna positioned in the needle.

In one aspect, the handle comprises a coolant manifold comprising a coolant supply connector configured to couple the coolant supply conduit to a supply pathway in the needle and a coolant return connector configured to couple the coolant return conduit to a return pathway in the needle.

In another aspect, the coolant manifold is positioned inside a handle casing adjacent a proximal end of the needle.

In another aspect, the probe assembly further comprises an analog-to-digital converter positioned in the handle.

In another aspect, the analog-to-digital converter converts an analog signal received from one or more sensors in the needle to a digital signal.

In another aspect, the analog-to-digital converter is electrically coupled to one or more sensors in the needle and to a low-voltage wire in the cable bundle.

In another aspect, the needle comprises one or more sensors configured to collect measurement data regarding operating conditions at the needle.

In another aspect, the one or more sensors are positioned on a flexible strip on an external surface of the needle.

In another aspect, the cable bundle comprises a conductive layer positioned externally to the cable and the coolant conduit.

In another aspect, the conductive layer is in thermal communication with both the power cable and the coolant supply conduit to conduct thermal energy from the power cable to the coolant supply conduit.

In another aspect, the coolant supply conduit is configured to guide coolant toward the microwave antenna and the coolant return conduit is configured to guide coolant away from the microwave antenna.

In another aspect, the coolant supply conduit, the coolant return conduit, and the power cable each contact each other and contact the conductive layer.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an isometric view of an example microwave ablation system in accordance with some embodiments of the present disclosure.
FIG. 2 is an isometric view of an example microwave ablation console that can be part of the microwave ablation system of FIG. 1.
FIG. 3 is a cross-sectional isometric view of an example cable bundle in accordance with some embodiments of the present disclosure.
FIG. 4 is a cross-sectional view of another example cable bundle in accordance with some embodiments of the present disclosure.
FIG. 5 is a cross-sectional view of another example cable bundle in accordance with some embodiments of the present disclosure.
FIG. 6 is a graphical illustration of a temperature profile of the cable bundle of FIG. 5 without a conductive layer during a simulated ablation procedure.
FIG. 7 is a graphical illustration of a temperature profile of the cable bundle of FIG. 5 with a conductive layer during a simulated ablation procedure.
FIG. 8 is a cross-sectional isometric view of an example cable bundle in accordance with some embodiments of the present disclosure.
FIG. 9 is a graphical illustration of a temperature profile of the cable bundle of FIG. 8 during a simulated ablation procedure.
FIG. 10 is a side view of an example cable bundle connected to coolant conduits using an endcap.
FIG. 11 is a cross-sectional side view of the example cable bundle and endcap of FIG. 10.
FIG. 12 is a magnified view of the endcap of FIG. 11.
FIG. 13 is a top view of the cable bundle of FIG. 10.
FIG. 14 is a cross-sectional isometric view of another example cable bundle in accordance with some embodiments of the present disclosure.
FIG. 15 is a graphical illustration of a temperature profile of the cable bundle of FIG. 14 during a simulated ablation procedure.
FIG. 16 is a cross-sectional view of another example cable bundle in accordance with some embodiments of the present disclosure.
FIG. 17 is a cross-sectional view of another example cable bundle in accordance with some embodiments of the present disclosure.
FIG. 18 is a cross-sectional view of another example cable bundle in accordance with some embodiments of the present disclosure.
FIG. 19 is a cross-sectional isometric view of another example cable bundle in accordance with some embodiments of the present disclosure.
FIG. 20 is a graphical illustration of a temperature profile of the cable bundle of FIG. 19 during a simulated ablation procedure.
FIG. 21 is a side view of an example ablation probe assembly in accordance with some embodiments of the present disclosure.
FIG. 22 is a side view of a corner portion of the ablation probe of FIG. 21 shown with part of the casing removed to show internal components.

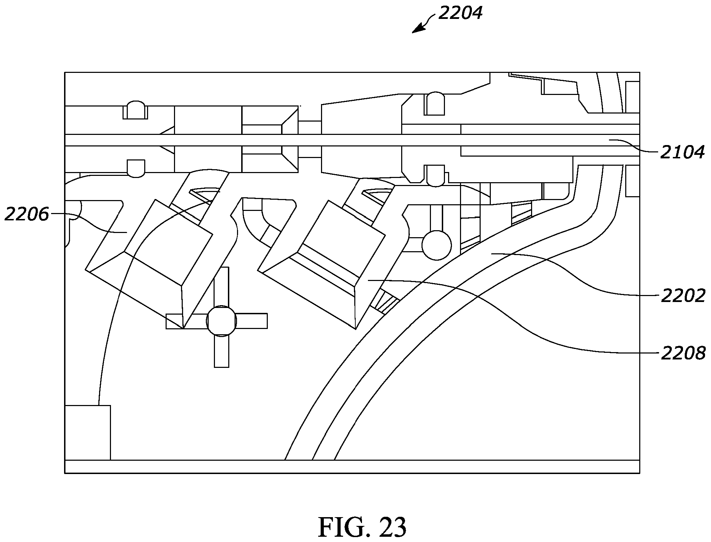

FIG. 23 is a magnified view of an example coolant manifold of FIG. 22.
FIG. 24 is a side view of an example ablation probe in accordance with some embodiments of the present disclosure.

Corresponding reference numerals indicate corresponding parts throughout the several views of the drawings.

### DETAILED DESCRIPTION

Example embodiments will now be described more fully with reference to the accompanying drawings. For purposes of the description hereinafter, it is to be understood that the embodiments described below may assume alternative variations and embodiments. It is also to be understood that the specific articles, compositions, and/or processes described herein are exemplary and should not be considered as limiting. In the description, relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

In the present disclosure the singular forms "a," "an," and "the" include the plural reference, and reference to a particular numerical value includes at least that particular value, unless the context clearly indicates otherwise. When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", "2-5", and the like. In addition, when a list of alternatives is positively provided, such listing can be interpreted to mean that any of the alternatives may be excluded, e.g., by a negative limitation in the claims. For example, when a range of "1 to 5" is recited, the recited range may be construed as including situations whereby any of 1, 2, 3, 4, or 5 are negatively excluded; thus, a recitation of "1 to 5" may be construed as "1 and 3-5, but not 2", or simply "wherein 2 is not included." It is intended that any component, element, attribute, or step that is positively recited herein may be explicitly excluded in the claims, whether such components, elements, attributes, or steps are listed as alternatives or whether they are recited in isolation.

In some embodiments of the present disclosure, an improved microwave ablation cable bundle is provided. The improved microwave ablation cable bundle may include one or more internal coolant bundles that can transport coolant through the cable to cool the power cable that delivers a microwave signal to the antenna of the ablation probe during operation. The coolant conduits may supply and return coolant in a coolant flow path through the cable and the ablation probe. The cable bundle may maintain the external surface of the cable bundle at an acceptable temperature to prevent burns or other hazards to users. Furthermore, the cable bundle may be structured so that it can bend or otherwise be easily routed from a microwave ablation console to a desired location in an ablation treatment environment.

The cable bundles of the present disclosure are improvements over existing or traditional cable structures. Existing cable structures may have no active cooling structures that actively cool the power cable of a microwave ablation cable. Such existing designs may include a large or thick outer shell or insulating layer. Such layers, however, often do not perform well and do not lower or maintain the outer surface of the cable at an acceptable temperature. Other additional protective features are used such as additional sheaths or additional covers in an attempt to shield or protect users from the hazards of the heated cable. These additional protective features are often ineffective, cumbersome, and/or aesthetically unacceptable. Still other existing cables may include a structure in which a power cable is concentrically surrounded by an outer coolant pathway. Such a cable-in-tube design has several drawbacks. This structure is difficult and/or complex to seal and can often lead to leaks or other problems. The propensity for leaks limits the fluid flows and pressures that can be achieved in cable-in-tube designs. The limitations on fluid flows and pressures can limit and/or prevent advantageous designs of slimmer needles or increased power levels of ablation needles because of the limitations to the cooling of the probe in cable-in-tube designs. The cable-in-tube design may also be difficult to bend or route to desired locations. The cable-in-tube design also limits the types and range of materials that can be used because the cable is surrounded by and/or in contact with the coolant fluid.

Existing cables and ablation needle assemblies are also limited as to their length due to the limitations of managing and/or removing the heat from the cables. Furthermore, the microwave generators may be limited in power to deliver sufficient microwave signals to the needle of existing designs. The cable bundles of the present disclosure overcome these limitations. The cable bundles of the present disclosure may achieve lengths of at least 10 feet in length. This length allows for more flexibility in the treatment environment because the ablation console may be positioned further away from the patient and other equipment in the treatment environment. The cooling functionality and configuration of the cable bundles of the present disclosure may allow such improved lengths to be achieved over the limited lengths of existing cable bundles. Furthermore, the improved designs of the cable bundles of the present disclosure allow more powerful microwave generators to be used (e.g., greater than 300W each) to achieve improved ablation performance at the extended lengths.

The present disclosure also provides improved ablation probes and improved ablation probe handles. The probes and handles of the present disclosure may be designed and/or adapted to be used in connection with the improved cable bundles described herein. Furthermore, the ablation probes and handles may have improved functionality such as integrated manifolds, analog-to-digital converters, as well as sensors to aid in the ablation process and improve performance over existing designs.

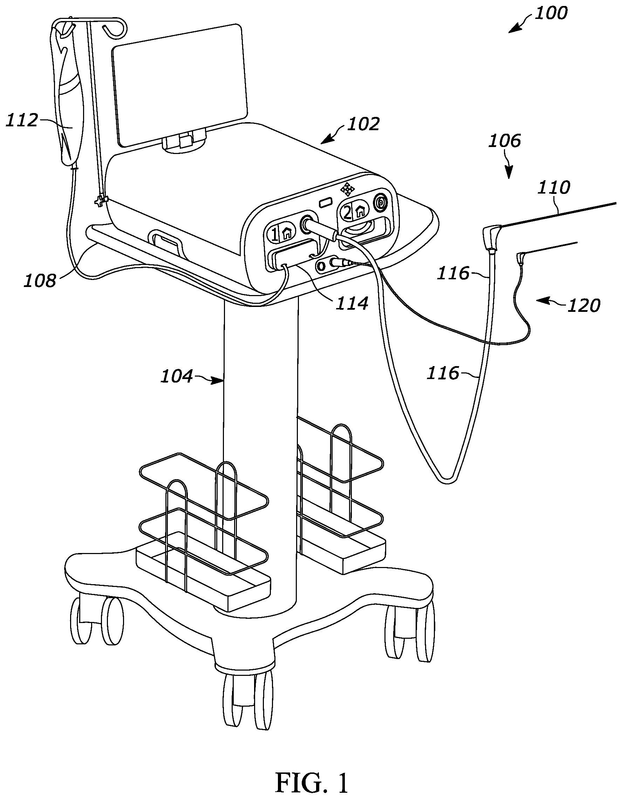

Referring now to FIG. 1, an example microwave ablation apparatus 100 is shown. The microwave ablation apparatus 100 may include a portable base unit or console 102, a cart 104, an ablation probe assembly 106, and a sensor probe assembly 120. The console 102 can be positioned on the cart 104. As will be further described, the console 102 may include a microwave generator and a coolant pump. In some embodiments, the ablation apparatus 100 may have a structure and functionality described in U.S. Patent Application No. TBD, titled PORTABLE ABLATION APPARATUS AND RELATED METHODS OF USE, filed on the same day as the present application to Varian Medical Systems, Inc. The contents of this application are incorporated by reference herein in its entirety. In various embodiments, the ablation apparatus 100 can be operated to perform ablation treatments by sending a microwave signal to an antenna in an ablation probe 110 via cable bundle 116. The antenna may deliver microwave energy to a target tissue at or near the ablation probe 110. The microwaves may induce an elevated temperature at the target tissue to destroy the target tissue.

During the ablation treatment, the ablation apparatus 100 may also deliver a coolant from a coolant receptacle 112 to the probe 110 using the coolant flow generator. The coolant cartridge 114 may be inserted into the front face of the console 102 and engage a pump to move the coolant from the coolant receptacle 112 to the probe 110 through the cable bundle 116. The movement of the coolant may absorb thermal energy from the cable and/or probe 110 effectively cooling the cable and/or portions of the probe 110.

As can be seen, the console 102 and the cart 104 can be easily moved in a hospital, treatment center, or other environment. The cart 104 may include a support surface such as a platform 108 that is positioned at an elevated position above the floor to allow access to the functionality of the system. The platform 108 may be positioned, for example, at a height approximately the same as a height of a patient. The patient that is undergoing an ablation treatment may be, for example, positioned on a bed of an imaging system. The imaging system may be a CT scanner, MRI machine, X-ray apparatus or other imaging device. With the console 102 positioned at a height approximately equal to that of the bed of the imaging system, the ablation treatment can be more easily performed. In addition, the console 102 can be moved next to the bed so that the length of the cables 116 that may be required to deliver microwave signals and/or coolant from the base unit to the probe 110 can be reduced over ablation systems that are more cumbersome or may need to be located at other positioned in the treatment environment. In some examples, the cables 116 may be at least 36 inches in length. In other examples, the cables 116 may be at least 10 feet in length. In other examples, other lengths may be used.

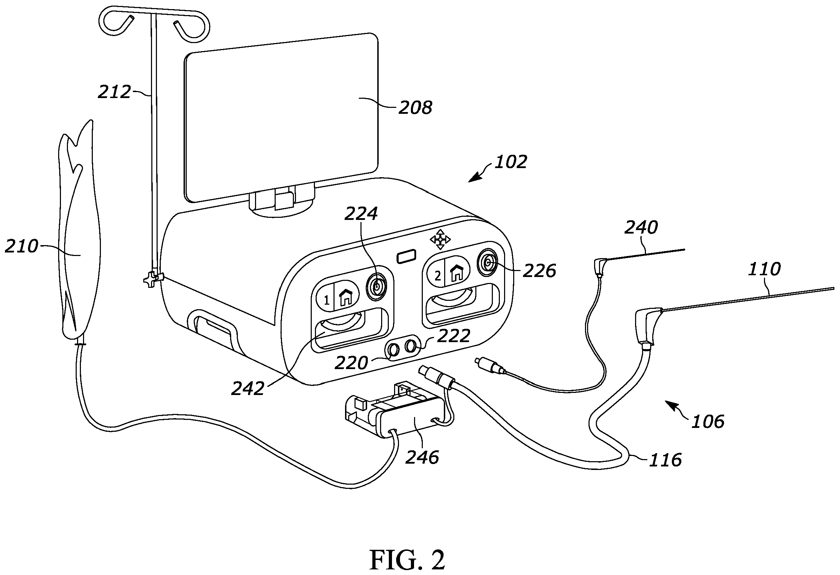

The console 102 can be portable in that it can be lifted and moved by one user. The console 102 can be removed from the cart 104 and be positioned on a table, counter, or other surface in a treatment or laboratory setting. The console 102 may include a housing 204 that is positioned around the inner components of the console 102. The housing 204 may include one or more walls that define an interior volume in which the inner components are located. As shown in FIG. 2, the console 102 may include a first microwave port 224, a second microwave port 226, a first sensor port 220, and a second sensor port 222. The console 102 may also include a display 208 to allow input from a user and to display one or more operating parameters of the console 102.

The first microwave port 224 and the second microwave port 226 can be similarly configured and can be an electrical connector to allow a mating connector to electrically couple the ablation probe assembly 106 to a respective microwave generator positioned inside the housing 204. In some examples, the first microwave port 224 may include a first marking and the second microwave port 226 may include a second marking. The first marking and the second marking may be different to allow a user to differentiate between the ports. The first marking and the second marking may include various suitable identifiers such as a color, a symbol, a letter, a number, a shape, a colored light or others. In some examples, the first marking and the second marking may include multiple identifiers from the previous list of identifiers. The first marking and the second marking can be used so as to guide an operator in the set-up and use of the console 102 to ensure that the proper connections are made by the operator.

The first microwave port 224 and the second microwave port 226 may be positioned in a front face 230 of the housing 204. This may allow a user to easily connect an ablation probe assembly 106 to the first microwave port 224 and/or the second microwave port 226. In other examples, the first microwave port 224 and the second microwave port 226 may be positioned at other locations in the housing 204 such as in the side walls, or top surface of the housing 204. While the example console 102 shown in FIG. 2 includes two microwave generators and two microwave ports, it should be appreciated that one generator and port may be included in some examples and more than two generators and ports may be included in other examples.

As further shown in this example, the console 102 may include a first sensor port 220 and a second sensor port 222 positioned on the front face 230. This positioning can allow an operator to easily connect one or more sensors to the console 102. As shown in FIG. 2, the ablation probe assembly 106 may include a sensor probe 240. The sensor probe 240 may be positioned at or near the target tissue in the patient during an ablation treatment to monitor and/or determine one or more parameters. For example, the sensor probe 240 may include one or more measurement points that can measure an impedance, temperature, moisture, density, or other characteristics of the patient. This measurement information can be transferred via a sensor cable 242 to the console 102. The sensor cable 228 may terminate at a sensor connector that can be connected to the console 102 at the first sensor port 220 and/or the second sensor port 222. This information can be sent to a processor, memory, or other element in the console 102 to be stored and/or displayed on the display 208. While the example base unit 102 includes two sensor ports 220, 222, it should be appreciated that the console 102 may include less than two sensor ports or more than two sensor ports in other examples.

The console 102 may also include two or more coolant flow generators. The coolant flow generators may be configured to move coolant through the ablation probe assembly 106 to dissipate thermal energy and prevent elements of the ablation probe assembly 106 from heating to undesirable levels. The coolant flow generators may each include a separate and independent pump that can be operated individually to cool the corresponding probe assembly 106. Various suitable pumps may be used such as a peristaltic pump.

The coolant flow generators may each be used through interaction with a corresponding coolant port. In the example shown, the console 102 includes a first coolant port 242 and a second coolant port 244. A complimentary cartridges 246 can be received in the first coolant port 242 and/or the second coolant port 244. In other examples, the coolant ports may be positioned in other regions of the console 102 such as in a side wall of the housing 204. The coolant cartridge 246 may include a cradle and tubing that permits the pump to interact with the coolant cartridge 246 to move coolant (e.g., saline) from the coolant receptacle 112 through the probe assembly 106. The coolant receptacle 112 can be supported on a hanger 212. The hanger 212 can be an upright hook or bar that suspends the coolant receptacle 112 in a position vertically above the coolant port of the console 102.

In the example shown, the console 102 includes two coolant flow generators. In other examples, the console 102 may include one coolant flow generator. In still other examples, the console 102 may include more than two coolant flow generators. The example shown in FIG. 2 also shows a single ablation probe assembly 106. It should be appreciated, however, that other ablation systems may include more than one ablation probe assembly 106. The number of ablation probe assemblies may correspond to the number of microwave generators, microwave ports, sensor ports, and/or coolant flow generators. In some embodiments, the coolant flow generators and the coolant ports 242, 244 may have a structure and functionality described in U.S. Patent Application No. TBD, titled PUMP AND CASSETTE SYSTEM FOR USE IN MICROWAVE ABLATION, filed on the same day as the present application to Varian Medical System, Inc. The contents of this application are incorporated by reference herein in its entirety.

The microwave ablation system 100 is designed to elevate the temperature of the target tissue in the patient. During such a treatment, heat may be generated elsewhere in the system. Various aspects of the microwave ablation system 100 manage or dissipate the heat to maintain acceptable temperatures levels of various components to prevent premature failure or damage to the components and/or to prevent hazards to operators. The console 102 may include an internal cooling structure to manage the temperature of the internal components of the console 102. Descriptions of example cooling systems are included in U.S. Patent Application No. TBD, titled CONSOLE ARCHITECTURE FOR MICROWAVE ABLATION UNIT, filed on the same day as the present application to Varian Medical Systems, Inc.

The structure of the ablation probe assembly 106 may also serve to cool and/or otherwise manage the thermal energy that is produced during operation of the microwave ablation system 100. As shown in FIG. 2, the ablation probe assembly 106 may include a cable bundle 116 that couples the ablation probe 110 to the console 102. The cable bundle 116 may include a coolant pathway that allows coolant (e.g., saline) from the receptacle 210 to be moved through the cable bundle 116 and through the probe 110.

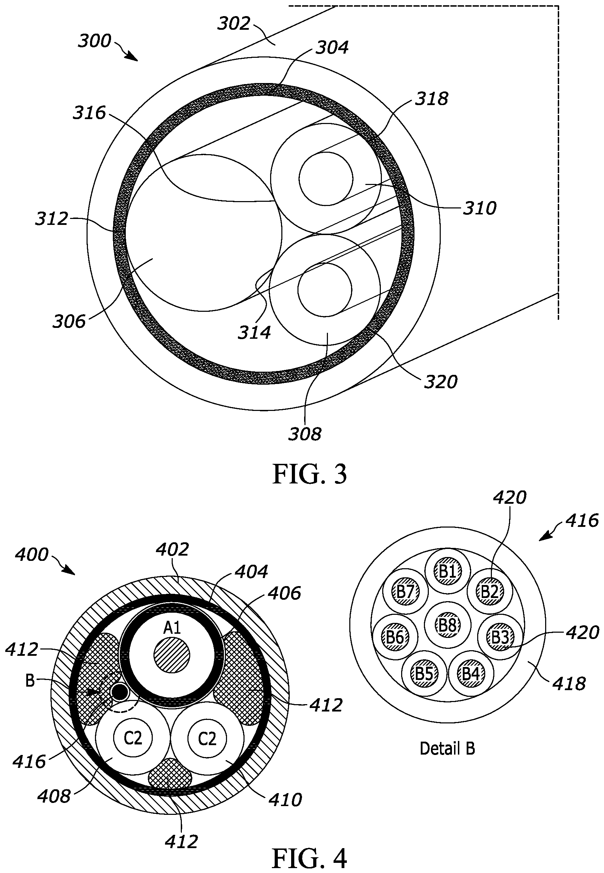

One example cable bundle 300 is shown in FIG. 3. In this example, the cable bundle 300 may include an outer layer 302, a conductive layer 304, a power cable 306, a coolant supply conduit 308 and a coolant return conduit 310. The power cable 306 may be any suitable wire or cable suitable to deliver a microwave signal from a microwave generator to the antenna in the ablation probe. In some examples, the power cable 306 is a coaxial cable with an internal conductor and concentrically positioned outer conductor. Such a coaxial cable may also include an outer insulating layer positioned radially outward of the outer conductor. The coolant supply conduit 308 and the coolant return conduit 310 may be positioned inside the cable bundle 300 and arranged next to or adjacent the power cable 306. The coolant supply conduit 308 and the coolant return conduit 310 may have a tubular shape with an internal opening to allow coolant to flow therethrough. The coolant supply conduit 208 and the coolant return conduit 310 may each be made of a suitable plastic tubing such as thermoplastic polyurethane (TPU) or the like.

The conductive layer 304 may be positioned radially outward of the power cable 306, the coolant supply conduit 308, and the coolant return conduit 310. The conductive layer 304 may be made of a thermally conductive material such as a metal like copper or aluminum. In other examples, other materials may be used. In one preferred example, the conductive layer 304 is made of a layer of braided aluminum or copper fibers. A braided construction can allow for improved flexibility, lower mass, and lower cost than other structures. In other examples, a solid layer, such as a foil, can alternatively be used.

The outer layer 302 is one or more layers of insulating material. Various materials such as foams or plastics may be used. Such materials may be extruded to form the elongated cylindrical shape. The outer layer 302 preferably has a smooth outer surface and compatibility with cleaners and other fluids that may be used to maintain a sterile and clean environment during an ablation treatment. The outer layer 302 is positioned radially outward of the conductive layer to form the outer surface of the cable bundle 300.

As can be seen in FIG. 3, the inner surface of the conductive layer 304 defines an internal cavity in the cable bundle 300. The power cable 306, the coolant supply conduit 308, and the coolant return conduit 310 may be positioned in the internal cavity of the cable bundle 300. The power cable 306 is positioned in a manner so that at least a portion of the outer surface of the power cable 306 contacts the conductive layer 304. As shown, the power cable 306 contacts the conductive layer at edge 312. Similarly, the coolant supply conduit 308 and the coolant return conduit 310 are each positioned in the internal cavity so that at least a portion of the outer surfaces of each of the conduits contact the inner surface of the conductive layer 304. As shown, the coolant supply conduit 308 contacts the conductive layer 304 at edge 320 and the coolant return conduit 310 contact the conductive layer 304 at edge 318.

The coolant supply conduit 308 may also contact the outer surface of the power cable 306 and the coolant return conduit 310 may contact the outer surface of the power cable 306. In this arrangement, the thermal energy may be transferred from the power cable to the coolant supply conduit 308 and/or to the coolant return conduit 310. Such transfer of thermal energy may also be assisted through conduction through the conductive layer 304. When the ablation procedure is being performed, the power cable 306 heats due to the microwave signal sent from the microwave generator to the antenna. At the same time, coolant is moved through the coolant supply conduit 308 and through the coolant return conduit 310. The heat generated in the power cable 306 can be transferred away from the cable bundle via the coolant in the coolant conduits. In this manner, the temperature of the cable bundle 300 can be maintained at a satisfactory temperature to reduce and/or minimize a risk of burn or injury.

Another example cable bundle 400 is shown in FIG. 4. In this example, the cable bundle 400 is similar to the cable bundle 300 previously described. The outer layer 402, the conductive layer 404, the power cable 406, the coolant supply conduit 408, and the coolant return conduit 410 may be configured as that previously described with respect to cable bundle 300. In this example, however, the cable bundle 400 may also include one or more fillers 412 and one or more low-voltage wire bundles 416.

The fillers 412 may be positioned in between the power cable 406, the coolant supply conduit 408, and/or the coolant return conduit 410 inside the conductive layer 404. The fillers 412 may be positioned, for example, in areas inside the conductive layer 404 that may otherwise collapse. The fillers 412 may maintain a circular cross-sectional shape of the cable bundle 400. The fillers 412 may be made of any suitable and/or compatible material. In some examples, the fillers 412 are made of nylon fibers. In other examples, other materials can be used.

The low-voltage wire bundle 416 can be positioned in other voids inside the conductive layer 404. The low-voltage wire bundle 416 can be positioned, for example, between the power cable 406, the coolant supply conduit 408, and/or the coolant return conduit 410 inside the conductive layer 404. The low-voltage wire bundle 416 can be provided to electrically couple sensors or other electrical contacts in the ablation probe to the ablation console 102. In the example shown in FIG. 4, the low-voltage wire bundle 416 may include eight individual low-voltage wires 420 positioned inside an insulating layer 418. In other examples, the low-voltage wire bundle 400 may include other quantities of low-voltage wires 420. In some examples, the low-voltage wire bundle 400 may include more than eight low-voltage wires 420. In other examples, the low-voltage wire bundle 400 may include less than eight low-voltage wires 420.

In this example, the wire bundle 400 includes one low-voltage wire bundle 416 positioned in a space between the power cable 406, the coolant supply conduit 408, and the filler 412. In other examples, the wire bundle 400 can include multiple low-voltage wire bundles 416 positioned at other positions inside the conductive layer 404. In still other examples, the wire bundle 400 may include more than one low-voltage wire bundle 416. In still other examples, the wire bundle 400 may not include any low-voltage wire bundles 416.

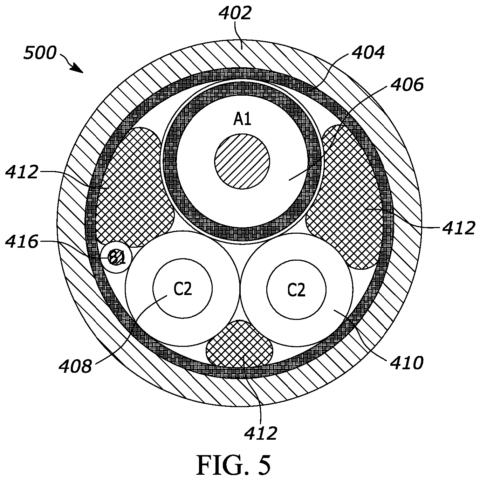

Another example cable bundle 400 is shown in FIG. 5. The cable bundle 500 is similar to the cable bundle 400 previously described. As shown, the cable bundle 500 includes the outer layer 402, the conductive layer 404, the power cable 406, the coolant supply conduit 408, the coolant return conduit 410, fillers 412, and the low-voltage wire bundle 416. The low-voltage wire bundle 416, in this example, is positioned at a different location than in the cable bundle 400. In this example, the low-voltage wire bundle 416 is positioned between the filler 412 and the coolant supply conduit 408 adjacent to the conductive layer 404. In this position, the low-voltage wire bundle 416 is spaced apart from the power cable 406 and distanced from the elevated temperatures of the power cable 406.

The cable bundles of the present disclosure with the conductive layer and the coolant conduits are improvements over existing microwave ablation cables and microwave ablation probes. The cable bundles of the present disclosure effectively reduce the operating temperatures of the microwave ablation cables to satisfactory levels to reduce and/or minimize burns or other hazards to the user. It can be desirable to minimize the operating temperatures of the microwave ablation cable during operation. A satisfactory temperature can be a temperature of less than or equal to 50°C. In other circumstances, a satisfactory temperature of a microwave ablation cable can be a temperature of less than or equal to 45°C. The cable bundles of the present disclosure can be operated to perform microwave ablations while being maintained at or below the previously described temperatures.

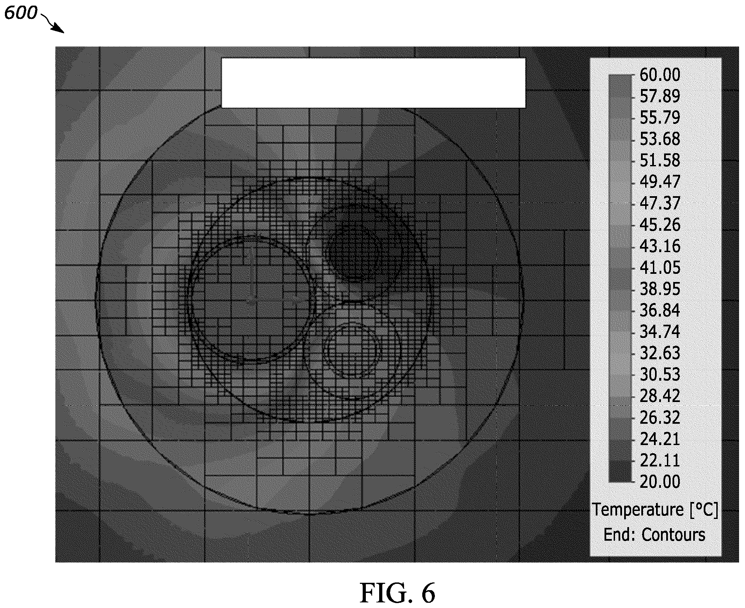

The conductive layer of the cable bundles of the present disclosure serves to distribute, conduct, and/or otherwise transfer heat away from the power cable and reduce the overall temperatures of the cable bundle more effectively than would otherwise occur in other cable bundles or existing designs. The cable bundles of the present disclosure were modeled with and without the conductive layer. FIG. 6 illustrates an operating temperature profile 600 of an example cable bundle without a conductive layer. The cable bundle includes an outer layer, a power cable, a coolant supply conduit, and a coolant return conduit but no conductive layer. As can be seen, the cable bundle still performs better than existing microwave ablation cables but temperatures of some portions of the cable bundle achieve temperatures at or around 60°C.

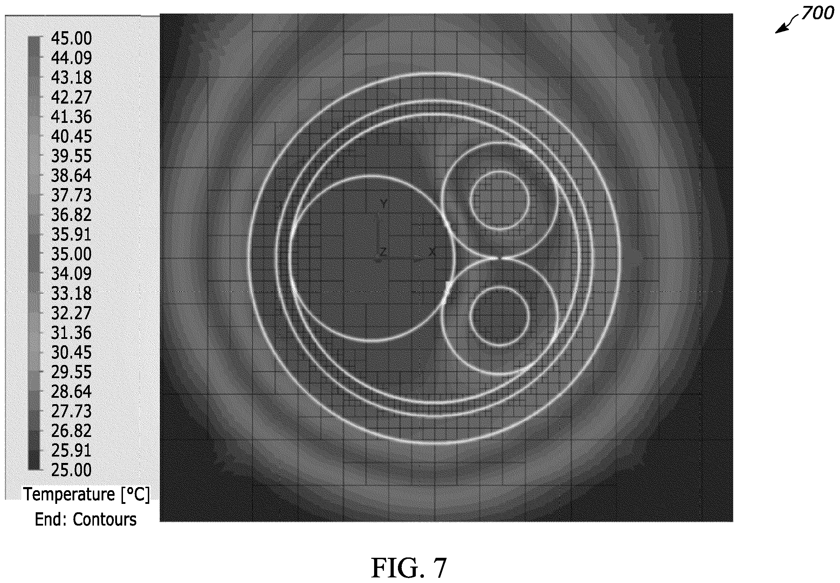

As shown in FIG. 7, a second operating temperature profile 700 is shown. In this example, a cable bundle is modeled that includes an outer layer, a power cable, a coolant supply conduit, a coolant return conduit and also a surrounding conductive layer. Under similar operating conditions as in FIG. 6, this cable bundle is able to more effectively distribute the thermal energy generated during operation and more heat is dissipated by the coolant and the ambient/surrounding air. The cable bundle in FIG. 7 achieves temperatures at or around 45°C. As shown, the cable bundle with the conductive layer can operate at acceptable temperature levels and at temperatures below the desired temperature levels discussed above.

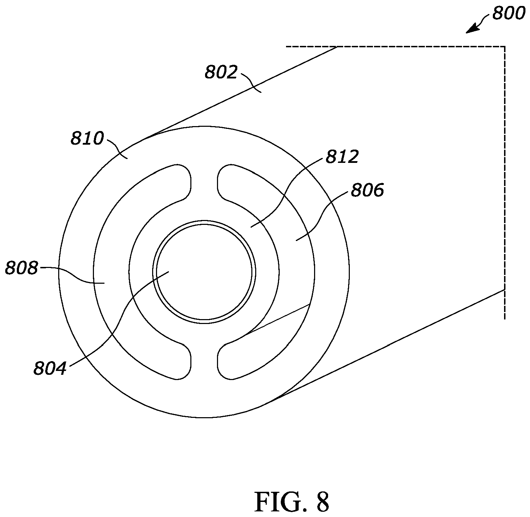

Turning now to FIG. 8, another example cable bundle 800 is shown. The cable bundle 800 can be operated similarly to the cable bundles discussed above to maintain safe operating temperatures and/or to dissipate the heat generated during operation of the microwave ablation probe assembly. In this example, the cable bundle 800 may include a shell portion 802 and a power cable 804. The power cable 804 can be configured as a coaxial cable to deliver the microwave signal from the microwave generator to an antenna in the ablation probe as previously described. The shell portion 802 may include an insulating layer 810 that defines an outer surface of the cable bundle 800. The shell portion 802 may also define one or more coolant lumens through which coolant may flow during operation of the microwave ablation assembly. In this example, the shell portion 802 defines a coolant supply lumen 806 and a coolant return lumen 808. The coolant supply lumen 806 may have a crescent shape or curved shape as shown and define a partial annular shape through which coolant may flow through the cable bundle. The coolant return lumen 808 may have a shape similar to the coolant supply lumen 806 and be positioned on an opposite side of the cable bundle 800. In other examples, the coolant supply lumen 806 and the coolant return lumen 808 may have different shapes such as circular cross-sectional shapes. In other examples, the shell portion may define a plurality of coolant supply lumens and a plurality of coolant return lumens.

In this example, the shell portion 802 also defines a sleeve 812. The sleeve 812 includes an opening in which the power cable 804 can be retained. In this example, the sleeve 812 is positioned at a center of the cable bundle 800. The coolant supply lumen 806 and the coolant return lumen 808 are each positioned radially outward of the sleeve 812 so that the power cable 804 is positioned in the center and the coolant supply lumen 806 and the coolant return lumen 808 are positioned on a concentric circle around the sleeve 812. In other examples, the shell portion may additionally define one or more sleeves in which low-voltage wire bundles may be retained.

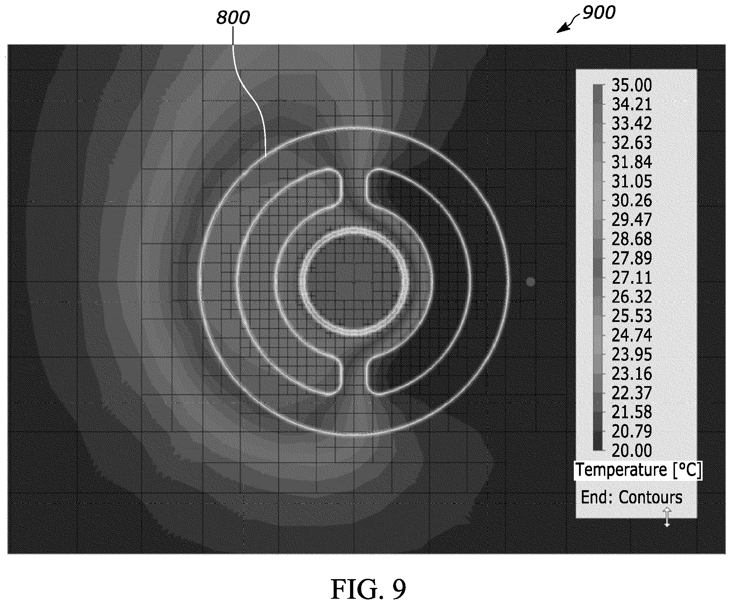

The cable bundle 800 was tested via modeling. A temperature profile 900 of the cable bundle 800 is shown in FIG. 9. As can be seen, the temperature levels of the cable bundle 800 are maintained at a temperature less than or equal to 35°C during operation of the microwave ablation probe assembly. The heat generated by the power cable is transferred to the coolant flowing through the coolant supply lumen and/or the coolant return lumen to maintain the temperature of the cable bundle at or below a safe operating temperature level.

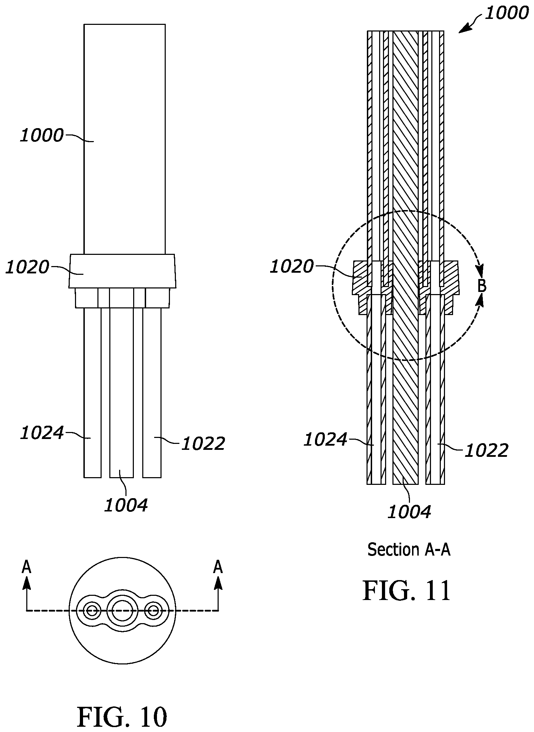

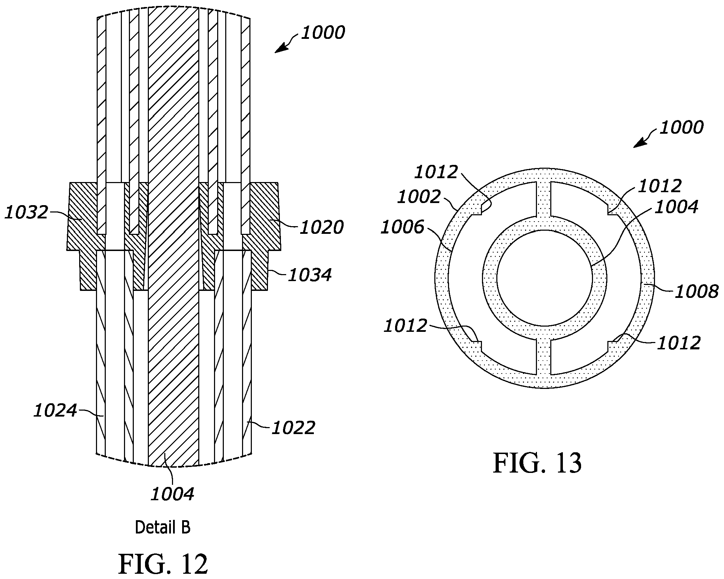

Another example cable bundle 1000 is shown in FIGs. 10-13. The cable bundle 1000 in this example is similar to the cable bundle 800 described above. A cross-section of the cable bundle 1000 is shown in FIG. 13. As shown, the cable bundle 1000 includes a shell portion 1002 that defines a power cable opening 1005, a coolant supply lumen 1006, and a coolant return lumen 1008. The configuration of the shell portion 1002 can allow a power cable 1004 to be positioned in the shell portion 1002 as shown in FIGs. 11 and 12. During operation, coolant may flow in the coolant supply lumen 1006 and the coolant return lumen 1008. In this example, the shell portion 1002 may also include one or more ribs 1012 that can project radially inward into the coolant supply lumen 1006 and/or the coolant return lumen 1008. The ribs 1012 can add some stiffness to the shell portion 1012 to reduce a likelihood that the cable bundle 1000 will fold or kink to prevent the flow of coolant in the lumens. In this example, the shell portion 1002 includes four ribs 1012 evenly spaced around a circumference of the inside surface of the shell portion 1002. In other examples, the shell portion 1002 may include more or less than four ribs. The ribs 1002 may also have other cross-sectional shapes that are different from the v-shaped ribs 1012 shown in FIG. 13. In other examples, the ribs 1012 may have rounded, arced, or semi-circular shapes. In other examples, the ribs 1012 may be oriented circumferentially instead of axially as shown, and may form repeating ring structures or a helix along the axial length of the shell portion.

As shown in FIGs. 10-12, the cable bundle 1000 may be joined to individual coolant tubing 1022, 1024 and/or to the power cable 1004 using an end cap 1020. The endcap 1020 may include transitions, mating connections, seals and/or other features to join the cable bundle 1000. The endcap 1020 may be used, for example, to operably connect the cable bundle to the microwave ablation console and/or elements of the microwave ablation systems previously described. The endcap 1020 may also be used at or in the handle of the ablation probe.

The endcap 1020 may include two sides. A first side 1032 (FIG. 12) may be used to couple the cable bundle 1000 to the endcap 1020. A second side 1034 may be used to couple individual wires, tubing, and/or conduits to the endcap 1020. The first side 1032 may have a female connector that has a profile complimentarily shaped to that of the cable bundle 1000. The second side 1034 may include individual separate openings to receive the power cable 1004, a coolant supply tubing 1022, and a coolant return tubing 1024. The endcap 1020 may serve to transition the flow of coolant from a circular tubing such as tubing 1022, 1024 to a non-circular coolant flow lumen in the cable bundle 1000, such as coolant supply lumen 1006 and/or coolant return lumen 1008. The cable bundle 1000 and/or the power cable 1004, the coolant supply tubing 1022, and/or the coolant return tubing 1024 may be fixed to the endcap 1020 using an adhesive, epoxy or other material. In other examples, barbs or interference fits may be used to connect the cable bundle and/or the cables and tubing to the endcap 1020.

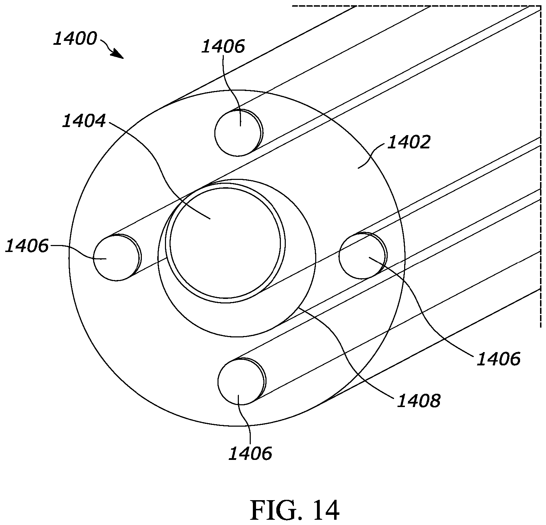

Turning now to FIG. 14, another example cable bundle 1400 is shown. In this example, the cable bundle 1400 may include one or more coolant lumens 1406 positioned in the shell portion 1402 of the cable bundle 1400. The shell portion 1402 may also include a central lumen 1408 that may be used to retain or guide the power cable 1404 to the microwave ablation probe. In this example, the central lumen 1408 has a diameter that is greater than the outer diameter of the power cable 1404. In other examples, the central lumen 1408 may have a diameter sized more closely to or interfering with the outer diameter of the power cable 1404.

In the example shown, two of the coolant lumens 1406 may be used as coolant supply lumens and two of the coolant lumens 1406 may be used as coolant return lumens. The lumens 1406 may be alternated around a circumference of the cable bundle 1400. For example, the lumen 1406 positioned at 3 o'clock may be a coolant supply lumen and the lumen 1406 positioned at 9 o'clock may be a coolant supply lumen and the lumen 1406 positioned at 12 o'clock may be a coolant return lumen and the lumen 1406 positioned at 6 o'clock may be a coolant return lumen. In this manner, the thermal energy transferred to the coolant in the lumens 1406 may be distributed around a circumference of the cable bundle 1400.

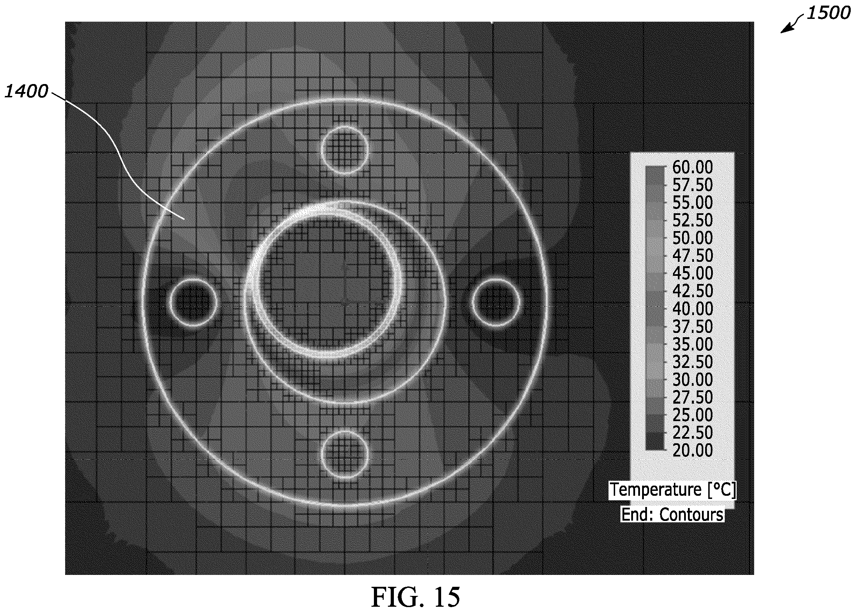

The cable bundle 1400 was tested via thermal modeling for its performance during operation. As shown in FIG. 15, the cable bundle 1400 is able to maintain satisfactory temperature ranges when the cable bundle is used during a microwave ablation procedure. As shown in the thermal profile 1500, the temperature of the outer surface of the cable bundle is maintained at or below at temperature of about 45°C. The thermal energy is dissipated by the shell portion 1402 and the surrounding ambient air and via the coolant flowing through the coolant lumens 1406.

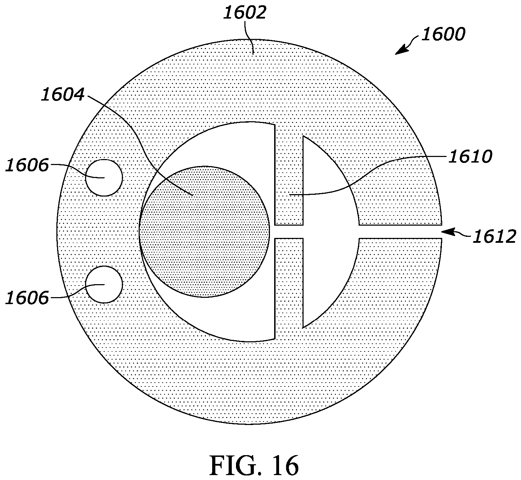

Another example cable bundle 1600 is shown in FIG. 16. In this example, the cable bundle 1600 includes a shell portion 1602. The shell portion 1602 can be formed such as by extrusion to have a cross-sectional profile as that shown. The shell portion 1602 may define an opening in which the power cable 1604 may be positioned. The shell portion 1602 may also define two or more coolant lumens 1606. In this example, the shell portion 1602 includes two coolant lumens 1606. The coolant lumens may serve as a coolant supply lumen and a coolant return lumen. The coolant lumens 1606 may operate as previously described to guide coolant to and from the ablation probe. In this example, the shell portion 1602 may also include an internal wall 1610 and a gap 1612. The gap 1612 may be an opening that extends through the shell portion from an outer surface through to the inner cavity. The gap 1612 may be formed or may be cut into the shell portion 1602. The gap 1612 may allow the power cable 1604 to be pushed into the inner cavity during assembly of the cable bundle 1600. While not shown, other wires, fillers, and/or conduits may also be installed into the inner cavity of the cable bundle 1600 through the gap 1612. The wall 1610 may be positioned in the inner cavity of the shell portion 1612 to retain or support the power cable 1604 in a desired position in the inner cavity. In this example, the wall 1610 captures and retains the power cable 1604 to a side of the inner cavity proximate to the coolant lumens 1606. In this manner, the thermal energy from the power cable 1604 can be transferred more effectively through the shell portion 1602 to the coolant in the coolant lumens 1606 than if the power cable were positioned elsewhere in the inner cavity.

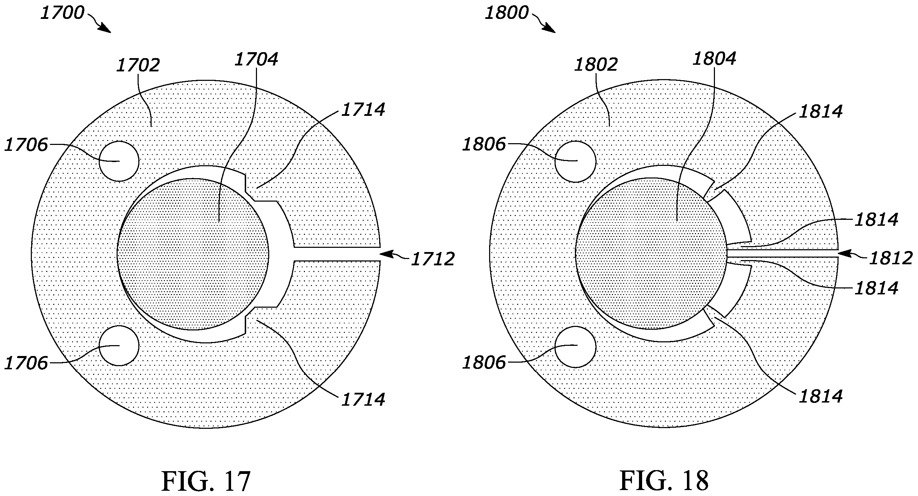

FIG. 17 shows yet another example cable bundle 1700. The cable bundle 1700 can operate and be used similarly to the cable bundle 1600 previously described. The cable bundle 1700 can be extruded or otherwise formed to have a circular outer profile and to define the inner cavity and the coolant lumens 1706. The power cable 1704 in this example is positioned in the inner cavity after being installed through the gap 1712. The ribs 1714 are positioned in the inner cavity and project inwardly from an inner surface of the shell portion 1702. The ribs 1714 can support or maintain the power cable 1704 in a position adjacent to the coolant lumens 1706 to facilitate effective transfer of thermal energy from the power cable to the coolant in the coolant lumens 1706.

FIG. 18 shows yet another cable bundle 1800. This example is similar to the cable bundle 1700 previously described. The shell portion 1802 of the cable bundle 1800 may define an inner cavity and one or more coolant lumens 1806. The shell portion 1802 also includes a gap 1812 to allow the power cable to be installed into the inner cavity. The shell portion 1802, in this example, includes multiple fingers 1814 that project inwardly from an inner surface of the shell portion 1802 in the inner cavity to retain and support the power cable 1804 in a desired position adjacent or proximate to the coolant lumens 1806.

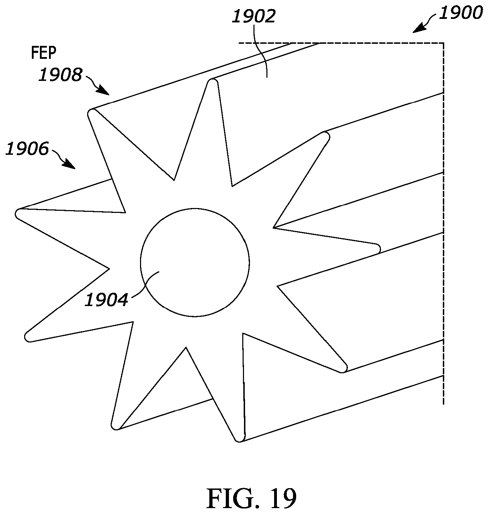

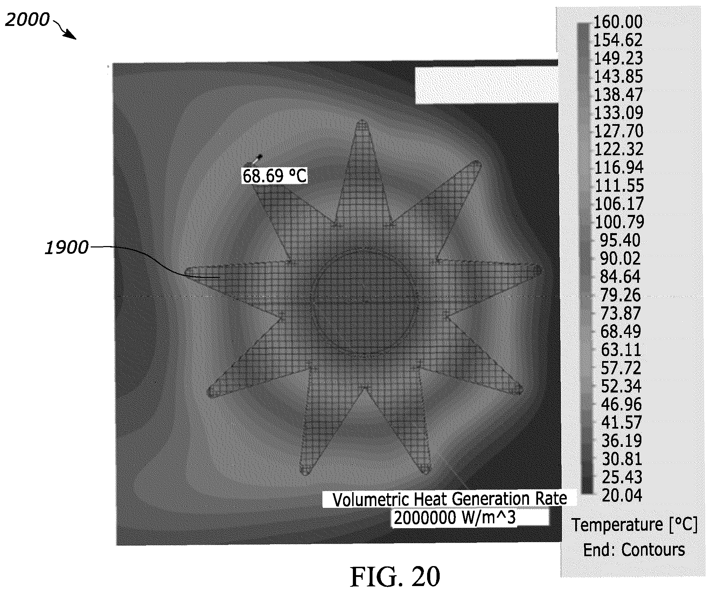

Referring now to FIGs. 19 and 20, another example cable bundle 1900 is shown. The cable bundle 1900 includes shell portion 1902 that may have various outer or cross-sectional profiles to allow heat transfer with the ambient environment. Unlike the previous examples with smooth or circular profiles, the shell portion 1902 in this example has a star cross-sectional shape. The cross-sectional shape includes multiple peaks 1908 and valleys 1906 around its circumference. In the example shown, the cross-sectional star shape includes nine peaks evenly spaced around the circumference. In other examples, the cross-sectional shape may have other shapes or profiles such as multiple fins, fingers, or other projections extending away from a center of the cable bundle 1900.

In the example shown, a power cable may be inserted in the central opening 1904 of the cable bundle 1900. In some examples, the cable bundle 1900 does not include active coolant lumens to cool the cable bundle. In other examples, the cable bundle 1900 can include one or more coolant lumens extending through one or more of the projections of the shell portion 1902. The projections of the shell portion 1902 include increased surface area compared to a smooth outer surface to allow ambient air to more effectively cool the cable bundle 1900.

A thermal profile 2000 of the cable bundle 1900 is shown in FIG. 20. As shown, the cable bundle 1900 may maintain a satisfactory temperature at an outer location at the peaks 1908 of the cable bundle 1900. The valleys 1906 may have higher temperatures but since these locations will likely not contact a user due to the extended peaks 1908, the cable bundle 1900 reduces a likelihood of burn or other hazard to a user.

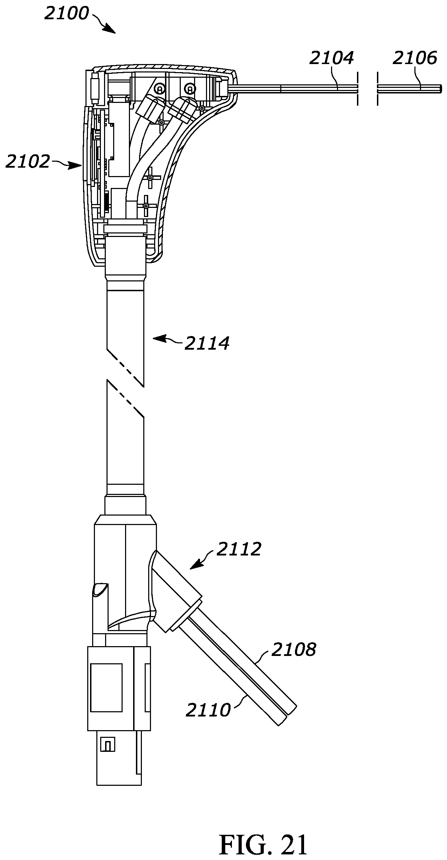

Referring now to FIG. 21, and example ablation probe assembly 2100 is shown. The probe assembly 2100, in this example, includes a handle 2102, a cable bundle 2114, and a needle 2104. The needle 2104 may extend away from the handle 2102 and terminate at a distal end 2106. The probe assembly 2100 may be coupled to the cable bundle 2114. The cable bundle 2114 may be structured as previously described and be coupled to a microwave ablation console. The probe assembly 2100 may include active cooling from the coolant that moves through the cable bundle 2114 as previously described. Such coolant may move through the handle 2102, through the cable bundle 2114, and through the needle 2104 to manage and/or control the temperatures of the handle 2102, the cable bundle 2114 and/or the needle 2104.

In the example shown, the cable bundle 2114 may include a coolant interface portion 2112. The coolant interface portion 2112 may allow coolant conduits to be fluidly connected between the cable bundle 2114 and the coolant cartridge 246. In this example, a coolant supply conduit 2108 and a coolant return conduit 2110 may be coupled to the coolant interface portion 2112. The coolant interface portion 2112 may include a quick connect fitting, barb fitting, push fitting, or other suitable connector to allow the coolant supply conduit 2108 and/or the coolant return conduit 2110 to be fluidly connected to the cable bundle 2114 at the coolant interface portion 2112. The coolant supply conduit 2108 and/or the coolant return conduit 2110 may extend from and/or be fluidly coupled to the coolant cartridge 246 as previously described. In some examples, the cable bundle 2114 and/or the coolant interface portion 2112 may include an endcap, such as endcap 1000 (FIG. 10), to transition from internal fluid conduits or internal coolant lumens to tubing or individual conduits. In another example, the coolant interface portion 2112 of the cable bundle 2114 may include a pass-through opening in the walls or shells of the cable bundle 2114 allowing continuous coolant conduits to enter and/or exit the cable bundle 2114. The continuous coolant conduits in such examples may be unbroken tubes extending from the coolant cartridge 246 though the cable bundle 2114 to the handle 2102.

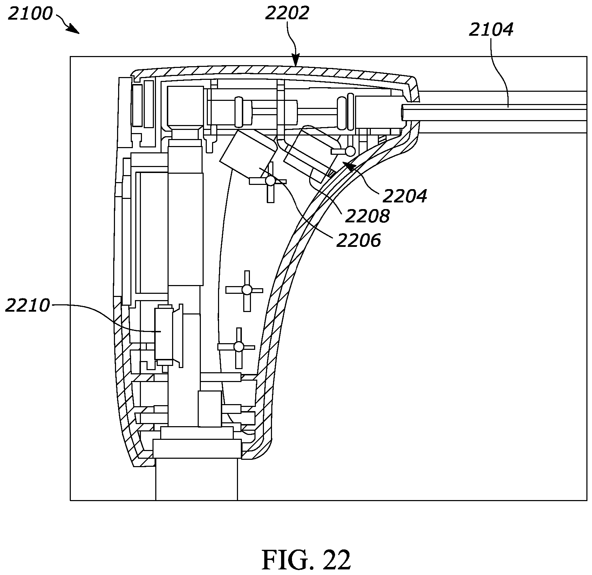

The handle 2102 of the probe assembly 2100 may include a casing that surrounds one or more internal components. The handle 2102 may include a corner portion 2202 (FIG. 22) that encloses various components of the ablation probe. The probe assembly 2100 may include a coolant manifold 2204. The coolant manifold 2204 may be positioned in the corner casing 2202. The coolant manifold 2204 may fluidly connect the coolant supply conduit to a coolant supply pathway in the needle 2104. The coolant manifold 2204 may also fluidly connect the coolant return conduit to a coolant return pathway in the needle 2104.

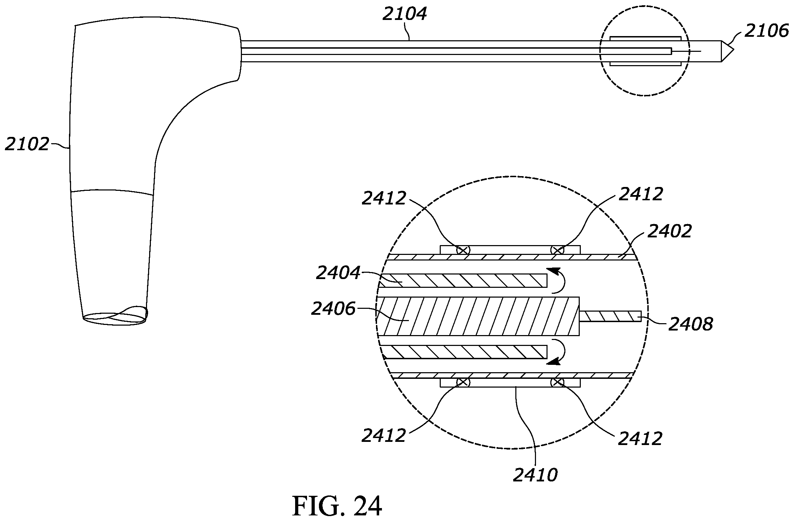

Referring now to FIG. 24, the needle 2104 may include an outer wall 2402 that defines an outer surface of the needle 2104. the power cable 2406 may be positioned inside the outer wall 2402 and may be positioned along a center axis of the needle 2104. The power cable 2406 may extend toward a distal end 2106 of the needle and have a microwave antenna 2408 positioned at or near the distal end 2106. The microwave signal that travels along the power cable 2406 may emit microwaves via the antenna 2408 to heat the target tissue during an ablation treatment. The emission of microwaves and/or the current from the microwave signal may heat the needle 2104 at or near the distal end 2106. To manage the temperature of the needle 2106, the coolant that moves through the cable bundle (as previously described) may flow into the needle through needle lumen 2404.

The coolant may flow toward the distal end 2106 through a coolant pathway defined between the inner surface of the needle lumen 2404 and the outer surface of the power cable 2406. After exiting an end of the needle lumen 2404, the coolant may flow back or away from the distal end 2106 of the needle 2104 via a return pathway defined between an inner surface of the outer wall 2402 and an outer surface of the needle lumen 2404. The flow of coolant may remove thermal energy from the needle 2104 to maintain the needle 2104 at a desired temperature.

At the proximate end of the needle 2104, the needle 2104 is connected to the coolant manifold 2204 inside the corner casing 2202. The return coolant pathway of the needle 2104 is fluidly connected in the coolant manifold 2204 to the return connector 2208. The supply coolant pathway of the needle 2104 is fluidly connected to the coolant manifold supply connector 2206. As shown, the return connector 2208 may include a sleeve to receive a coolant return conduit and the supply connector 2206 may include a sleeve to receive a coolant supply conduit. The coolant manifold 2204 is configured to allow a smooth and unimpeded transition of 90° from the cable bundle 2114 to the needle 2104. In other examples, the handle 2102 may have other orientations between the cable bundle 2114 and the needle 2104 that may be different from 90°. In such other configurations, the coolant manifold 2204 and/or the handle 2102 can be configured to allow the coolant conduits or tubing to be fluidly connected to the coolant pathways inside the needle 2104 and to transition a direction of the microwave signal cables from the cable bundle 2114 to the needle 2104.

The needle 2104 may also include a sensor strip 2410 (FIG. 24) that may be positioned on an external surface of the needle 2104. The sensor strip 2410 may include a plurality of measurement points 2412. The measurement points 2410 may be contacts, pads, sensors or other areas that can be used to collect measurement data regarding operating conditions at or around the needle 2104. For example, the measurement points may be electrical contact pads that can be used to measure impedance and/or temperature at or around the needle 2104. The sensor strip 2410 can collect information regarding conditions during an ablation procedure. The measurement data can be used by an operator and/or by the ablation console to control the operation of the ablation probe 2100 such as to adjust or control the microwave signal and/or to adjust or control the flow of coolant to the needle 2104.

The sensor strip 2410 may be configured as a sleeve of material that can be positioned over the needle 2104. The sensor strip 2410 may be shrink-wrapped into a desired position and/or secured using a suitable epoxy or adhesive. In other examples, the sensor strip 2410 may otherwise be deposited on the needle and/or formed into the needle using additive manufacturing or the like. The measurement points 2412 may be coupled to the cable bundle previously described, such as being connected to the low-voltage wire bundle. The measurement points 2412 may be routed through an analog-to-digital (AD) converter at some position before the signal is transferred to the ablation console for processing. In one example, the probe assembly 2100 may include an AD converter 2210 positioned in the handle 2102. In the example shown, the AD converter may be positioned in the corner casing 2202. The AD converter 2210 can convert the analog signals received from the sensor strip 2410 to digital signals. The digital signals may then be relayed to the ablation console for processing. The cable bundles and ablation probe assemblies of the present disclosure may be used in combination with ablation systems to perform ablation procedures that result in improved performance over existing systems and methods.

The example methods and apparatuses described herein may be at least partially embodied in the form of computer-implemented processes and apparatus for practicing those processes and/or the described functionality. The disclosed methods may also be at least partially embodied in the form of tangible, non-transient machine readable storage media encoded with computer program code. The media may include, for example, RAMs, ROMs, CD-ROMs, DVD-ROMs, BD-ROMs, hard disk drives, flash memories, or any other non-transient machine-readable storage medium, or any combination of these mediums, wherein, when the computer program code is loaded into and executed by a computer, the computer becomes an apparatus for practicing the method. The methods may also be at least partially embodied in the form of a computer into which computer program code is loaded and/or executed, such that, the computer becomes an apparatus for practicing the methods. When implemented on a general-purpose processor, the computer program code segments configure the processor to create specific logic circuits. The methods may alternatively be at least partially embodied in a digital signal processor formed of application specific integrated circuits for performing the methods.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

The following is a list of non-limiting illustrative embodiments disclosed herein:

Illustrative embodiment 1: A cable bundle comprising: a cable configured to deliver a microwave signal from a microwave generator to an antenna; a coolant conduit positioned adjacent the cable; a conductive layer positioned externally to the cable and the coolant conduit.

Illustrative embodiment 2: The cable bundle of illustrative embodiment 1, wherein the conductive layer is in thermal communication with both the cable and the coolant conduit to conduct thermal energy from the cable to the coolant conduit.

Illustrative embodiment 3: The cable bundle of any of the preceding illustrative embodiments, wherein the coolant conduit is configured to guide a flow of coolant through the cable bundle.

Illustrative embodiment 4. The cable bundle of any of the preceding illustrative embodiments, wherein the coolant conduit comprises a first coolant conduit and the cable bundle further comprises a second coolant conduit, the first coolant conduit configured to guide coolant toward the antenna and the second coolant conduit configured to guide coolant away from the antenna.

Illustrative embodiment 5: The cable bundle of illustrative embodiment 4, wherein the first coolant conduit, the second coolant conduit, and the cable each contact each other and contact the conductive layer.

Illustrative embodiment 6: The cable bundle of any of the preceding illustrative embodiments, wherein the conductive layer comprises a plurality of braided fibers.

Illustrative embodiment 7: The cable bundle of illustrative embodiment 6, wherein the plurality of braided fibers comprises aluminum or copper fibers.

Illustrative embodiment 8: The cable bundle of any of the preceding illustrative embodiments, further comprising one or more low-voltage wires positioned inside the conductive layer.

Illustrative embodiment 9: The cable bundle of any of the preceding illustrative embodiments, further comprising an outer layer positioned externally to the conductive layer, the outer layer comprising an insulating material.

Illustrative embodiment 10: The cable bundle of any of the preceding illustrative embodiments, further comprising a filler material positioned in one or more gaps between the coolant conduit and the conductive layer.

Illustrative embodiment 11: The cable bundle of any of the preceding illustrative embodiments, further comprising a console connector configured to electrically couple the cable to a microwave generator in a microwave ablation console.

Illustrative embodiment 12: The cable bundle of any of the preceding illustrative embodiments, wherein the coolant conduit is fluidly connected to a coolant cartridge configured to be received in a coolant port of a microwave ablation console.

Illustrative embodiment 13: The cable bundle of any of the preceding illustrative embodiments, having an axial length of at least 36 inches in length.

Illustrative embodiment 14: A microwave probe assembly comprising: a handle configured to operably connect to a cable bundle comprising a power cable, a coolant supply conduit and a coolant return conduit; a needle connected to the handle and extending away from the handle to a distal end; and a microwave antenna positioned in the needle.

Illustrative embodiment 15: The microwave probe assembly of illustrative embodiment 14, wherein the handle comprises a coolant manifold comprising a coolant supply connector configured to couple the coolant supply conduit to a supply pathway in the needle and a coolant return connector configured to couple the coolant return conduit to a return pathway in the needle.

Illustrative embodiment 16: The microwave probe assembly of illustrative embodiment 15, wherein the coolant manifold is positioned inside a handle housing adjacent a proximal end of the needle.

Illustrative embodiment 17: The microwave probe assembly of any of illustrative embodiments 14 to 16, further comprising an analog-to-digital converter positioned in the handle.

Illustrative embodiment 18: The microwave probe assembly of illustrative embodiment 17, wherein the analog-to-digital converter converts an analog signal received from one or more sensors in the needle to a digital signal.

Illustrative embodiment 19. The microwave probe assembly of any one of illustrative embodiments 17 or 18, wherein the analog-to-digital converted is electrically coupled to one or more sensors in the needle and to a low-voltage wire in the cable bundle.

Illustrative embodiment 20: The microwave probe assembly of any one of illustrative embodiments 15 to 19, wherein the needle comprises one or more sensors configured to collect measurement data regarding operating conditions at the needle.

Illustrative embodiment 21: The microwave probe assembly of illustrative embodiment 20, wherein the one or more sensors are positioned on a flexible strip on an external surface of the needle.

Illustrative embodiment 22: The microwave probe assembly of any one of illustrative embodiments 14 to 21, wherein the cable bundle comprises a conductive layer positioned externally to the cable and the coolant conduit.

Illustrative embodiment 23: The microwave probe assembly of illustrative embodiment 22, wherein the conductive layer is in thermal communication with both the power cable and the coolant supply conduit to conduct thermal energy from the power cable to the coolant supply conduit.

Illustrative embodiment 24: The microwave probe assembly of any one of illustrative embodiments 22 or 23, wherein the coolant supply conduit is configured to guide coolant toward the microwave antenna and the coolant return conduit is configured to guide coolant away from the microwave antenna.

Illustrative embodiment 25: The microwave probe assembly of any one of illustrative embodiments 22 to 24, wherein the coolant supply conduit, the coolant return conduit, and the power cable each contact each other and contact the conductive layer.

Illustrative embodiment 26: The microwave probe assembly of any of illustrative embodiments 14 to 25, further comprising a flow sensor position configured to determine one or more characteristics of a coolant flow.

## Claims

1. A cable bundle (300) comprising:
a cable (306) configured to deliver a microwave signal from a microwave generator to an antenna;
a coolant conduit (308) positioned adjacent the cable;
a conductive layer (304) positioned externally to the cable and the coolant conduit.

2. The cable bundle of claim 1, wherein the conductive layer is in thermal communication with both the cable and the coolant conduit to conduct thermal energy from the cable to the coolant conduit.

3. The cable bundle of claim 1 or claim 2, wherein the coolant conduit is configured to guide a flow of coolant through the cable bundle.

4. The cable bundle of any preceding claim, wherein the coolant conduit comprises a first coolant conduit and the cable bundle further comprises a second coolant conduit, the first coolant conduit configured to guide coolant toward the antenna and the second coolant conduit configured to guide coolant away from the antenna, optionally wherein the first coolant conduit, the second coolant conduit, and the cable each contact each other and contact the conductive layer.

5. The cable bundle of any preceding claim, wherein the conductive layer comprises a plurality of braided fibers, preferably wherein the plurality of braided fibers comprises aluminum or copper fibers.

6. The cable bundle of any preceding claim, further comprising one or more low-voltage wires positioned inside the conductive layer, and/or an outer layer positioned externally to the conductive layer, the outer layer comprising an insulating material, and/or a filler material positioned in one or more gaps between the coolant conduit and the conductive layer, and/or having an axial length of at least 36 inches in length.

7. The cable bundle of any preceding claim, further comprising a console connector configured to electrically couple the cable to a microwave generator in a microwave ablation console.

8. The cable bundle of any preceding claim, wherein the coolant conduit is fluidly connected to a coolant cartridge configured to be received in a coolant port of a microwave ablation console.

9. A microwave probe assembly comprising:
a handle configured to operably connect to a cable bundle comprising a power cable, a coolant supply conduit and a coolant return conduit;
a needle connected to the handle and extending away from the handle to a distal end; and
a microwave antenna positioned in the needle.

10. The microwave probe assembly of claim 9, wherein the handle comprises a coolant manifold comprising a coolant supply connector configured to couple the coolant supply conduit to a supply pathway in the needle and a coolant return connector configured to couple the coolant return conduit to a return pathway in the needle.

11. The microwave probe assembly of claim 9 or claim 10, wherein the coolant manifold is positioned inside a handle housing adjacent a proximal end of the needle.

12. The microwave probe assembly of any of claims 9 to 11, further comprising an analog-to-digital converter positioned in the handle, preferably wherein the analog-to-digital converter converts an analog signal received from one or more sensors in the needle to a digital signal, and/or wherein the analog-to-digital converted is electrically coupled to one or more sensors in the needle and to a low-voltage wire in the cable bundle.

13. The microwave probe assembly of any of claims 9 to 12, wherein the needle comprises one or more sensors configured to collect measurement data regarding operating conditions at the needle.

14. The microwave probe assembly of claim 13, wherein the one or more sensors are positioned on a flexible strip on an external surface of the needle.

15. The microwave probe assembly of any of claims 9 to 14, wherein the cable bundle comprises a conductive layer positioned externally to the cable and the coolant conduit, optionally wherein the conductive layer is in thermal communication with both the power cable and the coolant supply conduit to conduct thermal energy from the power cable to the coolant supply conduit, and/or wherein the coolant supply conduit is configured to guide coolant toward the microwave antenna and the coolant return conduit is configured to guide coolant away from the microwave antenna, and/or wherein the coolant supply conduit, the coolant return conduit, and the power cable each contact each other and contact the conductive layer.
